# EUROPEAN PATENT APPLICATION

(11) **EP 1 304 134 A1**
(43) Date of publication of application: **23.04.2003**
(21) Application number: 01947792.6
(22) Date of filing: 29.06.2001
(51) Int. Cl.: A61M 35/00

(54) **AUXILIARY DEVICE FOR STICKING PASTING AGENT**

(30) Priority: 30.06.2000 JP 2000199371; 30.06.2000 JP 2000199385
(71) Applicant: HISAMITSU PHARMACEUTICAL CO. INC., Tosu-shi, Saga-ken 841-0017 (JP)
(72) Inventor: HIRASHIMA, Nobuchika, c/o HISAMITSU PHARM. CO. INC, Tokyo 141-0031 (JP); MAEDA, Eiji, c/o HISAMITSU PHARMACEUTICAL CO. INC., Tokyo 141-0031 (JP); SHOHO, Kouki, c/o HISAMITSU PHARMACEUTICAL CO. INC, Tosu-shi, Saga 841-0017 (JP)
(74) Representative: Grünecker, Kinkeldey, Stockmair & Schwanhäusser Anwaltssozietät
(86) International application number: JP0105639
(87) International publication number: WO02002177

(57) **Abstract**

An auxiliary implement 10 for patch application is an auxiliary implement for applying a patch which is to be used to the desired area, which comprises a backing 2 having a surface larger than the patch, wherein on the upper surface of the backing serving as a patch holding surface for temporarily and peelably holding the patch there are provided an adhesive surface portion 3f of an adhesive layer 3 which is coated with a pressure-sensitive adhesive and a non-adhesive surface portion composed of the upper surface of the backing which is not coated with the adhesive layer and the upper surface 4f of a coating material 4, and at least part of the outer edge portion of the patch holding surface constitutes the non-adhesive surface portion which is easy to hold between the fingers.

## Description

### Technical Field

The present invention relates to an auxiliary implement for patch application.

### Background Art

Backings in the conventional patches had appropriate hardness and thickness and also appropriate flexibility. However, the ability to adhere closely to the affected area and a feeling of physical disorder after application have recently started giving concerns. Accordingly, a marked trend toward decreasing the thickness of the backing has been demonstrated. As a result, the strength and flexibility of the patch itself became insufficient, the patch lacked firmness, for example, when a peeling sheet attached to the adhesive surface of the patch containing a medically effective component was peeled off, the patch was very difficult to grasp, and the application operation required much labor. Furthermore, the patches lacking firmness prior to application to the affected area caused the following problems: the adhesive surfaces were entangled with each other and could not be reused, wrinkles appeared in the patch during application, making it impossible to cover the affected area completely and decreasing the percutaneous absorption ratio of medically effective components, and the like.

Accordingly, a variety of members for increasing the strength and flexibility of patches and applying the patches smoothly and evenly to the affected area and patches equipped with such members have heretofore been suggested.

For example, Japanese Utility Model Application Laid-open No. S56-57235 suggested a patch of the type in which a peeling sheet peelably pasted on the rear surface of the patch backing was provided as the above-mentioned member and the peeling sheet was peeled off from the backing after the patch has been pasted onto the affected area. Furthermore, Japanese Patent Application Laid-open No. S62-77314 suggested a medical patch using a highly flexible film-like substrate as a backing, this patch being of the type in which a support member sufficiently bendable to follow the shape deformation of the film-like substrate and provided with splits for smooth peeling from the backing after application was peelably pasted on the rear surface of the patch backing.

Further, Japanese Patent Application Laid-open No. S64-16719 suggested a patch of the type in which a support implement for a member for external application having weak adhesive capability was pre-pasted onto the rear surface of the patch backing. Japanese Patent Application Laid-open No. H10-290820 suggested an auxiliary implement for application which comprised a frame with an inner edge larger than the outer edge of a patch and a protruding portion disposed on the inner edge. Japanese Patent Application Laid-open No. H11-349476 also suggested a medical patch equipped with a support member in which a flexible substrate was peelably pasted on the rear surface of the patch backing.

### Disclosure of the Invention

However, the above-described conventional members for facilitating the application of a patch composed of a thin backing were discarded after each usage and could not be used repeatedly with a plurality of patches. Furthermore, obviously the problem of the adhesive surfaces of the patch being entangled with each other in the process of application and the problem of wrinkles appearing in the patch applied to the affected area could not be fully resolved.

Those problems were encountered especially often when elderly people were using patches comprising thin backings, and such patches comprising thin backings could hardly be considered as fully satisfying the requirements of the aging society.

The present invention was created with regard to the above-described problems of conventional technology, and it is an object of the present invention to provide an auxiliary implement for patch application which makes it possible to apply a patch smoothly and evenly to the affected area and which can be used repeatedly.

The inventors have conducted a comprehensive research to attain the aforesaid object and have found out that when a patch comprising a thin backing is used, the patch can be easily handled during application operation and entanglement of the adhesive surfaces of the patch to each other and origination of wrinkles can be fully prevented by using a member composed of a backing with a surface larger than that of the patch and the appropriate strength and flexibility as an auxiliary implement for application operation, integrating the patch with the auxiliary implement prior to application to the affected area, and using the patch upon such an augmentation of strength and flexibility thereof.

The inventors have also found out that providing a portion with appropriate adhesive properties and portion having no adhesive properties on the surface of the backing of the auxiliary implement makes it possible to apply the patch easier and more reliably to the desired area and to use the auxiliary implement repeatedly with a large number of patches.

Thus, the auxiliary implement for patch application in accordance with the present invention is an auxiliary implement for applying a patch which is to be used to the desired area and comprises a backing having a surface larger than the patch. On one surface of the backing, there are provided an adhesive surface portion which is coated with a pressure-sensitive adhesive and serves for temporarily and peelably holding the patch and a non-adhesive surface portion, wherein at least part of the outer edge portion of the above-mentioned surface is the non-adhesive surface portion.

With such an auxiliary implement for patch application (hereinbelow referred simply as "auxiliary implement"), prior to applying the patch to the affected area, the patch which is to be used can be temporarily held in advance in a state in which it is wrinkle-free evenly spread on the surface of the backing of the auxiliary implement, this surface being coated with the pressure-sensitive adhesive (hereinbelow referred to as "patch holding surface"). Because the patch is thus integrated with the auxiliary implement, the strength or flexibility thereof are adjusted to a level providing for easy handling during application operation. Therefore, the patch can be smoothly carried to the desired application area, while preventing the problem of partial bonding of the adhesive surfaces of the patch to each other caused by the patch bending during application operation. Moreover, the patch can be applied evenly and without wrinkles.

Further providing an adhesive surface portion and a non-adhesive surface portion on the patch holding surface of the backing of the auxiliary implement and adjusting the adhesive strength of the pressure-sensitive adhesive coated on the adhesive surface portion and the surface area ratio of the adhesive surface portion and non-adhesive surface portion makes it possible to adjust the adhesive strength between the rear surface of the patch and the patch holding surface. Therefore, the auxiliary implement can be used repeatedly with a large number of patches, without being discarded. Moreover, the problems of partial peeling of the patch held on the patch holding surface during application operation or complete peeling and separation can be prevented.

Because a non-adhesive surface portion is formed on at least a part of the outer edge portion of the patch holding surface, the user of the patch can conduct the application operation in an easy manner by holding this portion between the fingers. Thus, because the patch holding surface has a surface area larger than that of the patch, if the outer edge portion of the patch holding surface is such that it can be held between the fingers, then the operation can be conducted without the fingers touching the patch fixed to the patch holding surface during application operation. Moreover, if the operation is conducted by holding between the fingers the part of the outer edge portion where the non-adhesive surface portion was formed, the pressure-sensitive adhesive does not adhere to the finger tips. Therefore, the application operation can be conducted in an easy manner. In addition, if the pressure-sensitive adhesive doest not adhere to the finger tips, the decrease in adhesive strength of the adhesive surface portion in repeated usage can be substantially prevented and the service life of the auxiliary implement can be extended.

Furthermore, the auxiliary implement for patch application in accordance with the present invention is preferably further provided with a detachable peeling sheet protecting the adhesive surface portion on the patch holding surface. With such a configuration, dust or the like cannot adhere to the adhesive surface portion of the patch holding surface when the auxiliary implement is stored. Therefore, the decrease in the adhesive strength of the adhesive surface portion is effectively prevented and the number of repeated usages can be further increased.

When a peeling sheet is provided, the peeling sheet is preferably processed so that it can be easily peeled off from the auxiliary implement during utilization. For example, the so-called back splits of linear, S-like wavy, or saw-like shape may be provided in the central part of the peeling sheet attached to the patch holding surface of auxiliary implement. In such a case, the peripheral portion of the back split can be raised from the patch holding surface by slightly curving the auxiliary implement during usage, thereby facilitating peeling. Therefore, by holding this portion between the fingers, the user can easily peel off the peeling sheet from the auxiliary implement.

The back splits maybe the so-called stitch-like slits or complete slits completely separating the peeling sheet in advance. Furthermore, a folded portion that can be held between the fingers may be provided in advance in the portion of the back splits of the peeling sheet. In such a case, because the folded portion of the peeling sheet is not in contact with the patch holding surface, it can be easily held between the fingers, without adhesion of the pressure-sensitive adhesive and the peeling sheet can be peeled off from the auxiliary implement in an easy manner by raising and holding the folded portion of the peeling sheet between the fingers, even without curving the auxiliary implement.

Further, in the auxiliary implement for patch application in accordance with the present invention, the adhesive surface portions or non-adhesive surface portions may have a stripe-like, grid-like, or spot-like shape. In such a case, the adhesive surface portions or non-adhesive surface portions can be orderly disposed on the patch holding surface. Therefore, portions with and without tight adhesion between the rear surface of the patch backing and the patch holding surface can be formed with good balance.

As a result, when the patch is held on the patch holding surface, a state with a wrinkle-free even spread can be obtained easier. Moreover, with such a configuration, the adhesive strength between the patch holding surface and the rear surface of the patch backing is easier to apply almost uniformly over the entire rear surface of the patch backing. Therefore, when the patch holding surface is peeled off from the rear surface of the patch backing after the patch has been applied to the affected area, the auxiliary implement alone can be smoothly peeled off without causing a problem of partial peeling of the patch from the affected area.

Further, in the auxiliary implement for patch application in accordance with the present invention, the ratio of the surface area of the adhesive surface portion to the entire surface area of the patch holding surface is preferably 20-40%. In such a case, the adhesive strength between the patch holding surface and the rear surface of the patch backing can be easier adjusted to a level at which the patch prior to application to the affected area can be reliably held on the patch holding surface and the auxiliary implement can be smoothly peeled off from the patch after application to the affected area. If the ratio of the surface area of the adhesive surface portion to the entire surface area of the patch holding surface is less than 20%, the above-mentioned adhesive strength is insufficient, holding the patch reliably on the patch holding surface tends to become more difficult, and applying it to the affected area also tends to become more difficult. On the other hand, if the ratio exceeds 40%, problems associated with partial or complete peeling of the patch from the affected area when the auxiliary implement is peeled off from the patch after application to the affected area and appearance of wrinkles on the patch tend to become more probable. From the above-described standpoint, it is more preferred that the ratio of the surface area of the adhesive surface portion to the entire surface area of the patch holding surface be 25-35%.

Furthermore, in the auxiliary implement for patch application in accordance with the present invention, when the adhesive surface portions or non-adhesive surface portions have the above-mentioned stripe-like, grid-like, or spot-like shape, it is preferred that the adhesive strength of the adhesive surface portions based on the measurement test according to the probe tack test specified by JIS Z 0237-1991 be 4.0-7.5 N. In particular, it is even more preferred that the above-mentioned adhesive strength be 4.0-7.5 N when the adhesive surface portions or non-adhesive surface portions have above-mentioned stripe-like, grid-like, or spot-like shape and the ratio of the surface area of the adhesive surface portion to the entire surface area of the patch holding surface is 20-40%. In such a case, the adhesive strength between the patch holding surface and the rear surface of the patch backing can be more easily adjusted to a level at which a series of application operations can be conducted without inconveniences. In particular, if the adhesive strength of the adhesive surface portion is no less than 4.0 N, the auxiliary implement can be repeatedly used with a large number of patches.

If the adhesive strength is less than 4.0N, the adhesive strength is insufficient and it becomes more difficult to use the auxiliary implement repeatedly with a large number of patches. On the other hand, if the adhesive strength exceeds 7.5 N, problems associated with partial or complete peeling of the patch from the affected area when the auxiliary implement is peeled off from the patch after application to the affected area tend to become more probable. From the above-described standpoint, in case of the above-described configuration, it is more preferred that the adhesive strength of the adhesive surface portion be 4.5-7.0 N.

The "adhesive strength of the adhesive surface portion" as referred to in the present specification is an adhesive strength of the adhesive surface portion of the patch holding surface of the auxiliary implement measured by a measurement method basically following the probe tack test specified in section 5.3 of JIS Z 0237-1991, except that the contact speed and peeling speed area 5 mm/sec.

In the auxiliary implement for patch application in accordance with the present invention, the entire outer edge portion of the patch holding surface may be the non-adhesive surface portion and the entire zone on the inside of the non-adhesive surface portion may be the adhesive surface portion. With such a configuration, because the entire outer edge portion of patch holding surface is a non-adhesive surface portion, the non-adhesive surface portion, which is easy to hold between the fingers, can be held and the application operation can be conducted more smoothly even without paying special attention thereto when the auxiliary implement is handled. Furthermore, in this case, too, the adhesive strength between the patch application surface and the rear surface of the patch backing can be adjusted to a level at which the application operation can be conducted smoothly, by adjusting the adhesive strength of the pressure-sensitive adhesive.

Further, with the above-described configuration, the adhesive strength of the adhesive surface portion based on the measurement test according to the probe tack test specified by JIS Z 0237-1991 is preferably 1.7-4.0 N. In such a case, the adhesive strength between the patch holding surface and the rear surface of the patch backing can be more reliably adjusted to a level at which a series of application operations can be conducted without inconveniences. In particular, in case of such a configuration, if the adhesive strength of the adhesive surface portion is no less than 1.7N, the auxiliary implement can be repeatedly used with a large number of patches.

If the adhesive strength is less than 1.7 N, the adhesive strength is insufficient and it becomes more difficult to use the auxiliary implement repeatedly with a large number of patches. On the other hand, if the adhesive strength exceeds 4.0 N, problems associated with partial or complete peeling of the patch from the affected area when the auxiliary implement is peeled off from the patch after application to the affected area tend to become more probable. From the above-described standpoint, in case of the above-described configuration, it is more preferred that the adhesive strength of the adhesive surface portion based on the measurement test according to the probe tack test specified by JIS Z 0237-1991 be 2.0-3.5 N.

In the auxiliary implement for patch application in accordance with the present invention, the peeling force acting between the adhesive surface portion and the rear surface of the patch backing based on the measurement test according to the 180-degree peeling method specified by JIS Z 0237-1991 is preferably no more than 1.0 N/20 mm. In such a case, the adhesive strength between the patch holding surface and the rear surface of the patch backing can be more reliably adjusted to a level at which a series of application operations can be conducted without inconveniences. If the peeling force exceeds 1.0 N/20 mm, problems associated with partial or complete peeling of the patch from the affected area when the auxiliary implement is peeled off from the patch after application to the affected area tend to become more probable.

In accordance with the present invention, "the peeling force acting between the adhesive surface portion and the rear surface of the patch backing" indicates the adhesive strength of the patch holding surface of the auxiliary implement with respect to the rear surface of the patch backing that was measured by a measurement method basically following the 180-degree peeling method using a tensile test machine of a constant speed tension type specified by JIS Z 0237-1991 8.3.1(1) (1.2) (a).

Thus, first, a patch (25 x 50 mm) is placed, with the adhesive surface thereof facing down, on a cleaned test plate, one end of the patch in the lengthwise direction is brought in line with one end of the test plate, the patch holding surface of a sheet-like sample (20 x about 100 mm, thickness 100-200 µm) of the auxiliary implement for patch application is applied to the rear surface of the patch backing so that the sample is brought into the center of the patch in the widthwise direction thereof, and the remaining portion (20 x about 50 mm) of the sample is left unattached.

The portion (20 x 50 mm) of the sample that has been pasted on the rear surface of the patch backing secured on the test plate is then further bonded under pressure by a single-cycle reciprocal movement of a roller at a speed of about 300 mm/min from above the sample. The free portion of the sample is 180-degree folded for 20-40 min after pressure bonding. Paper (20 x 100 mm, thickness 100 µm) is then pasted onto the portion (20 x about 50 mm) of the patch holding surface of the folded sample and the remaining portion (20 x about 50 mm) of the paper is left unattached. The portion of the sample that has been pressure bonded to the rear surface of the patch backing is then peeled about 5 mm from the fold position, the free portion of the paper and the test plate are inserted respectively in the upper and lower clamps of the tensile test machine, and the sample is peeled off from the rear surface of the patch backing at a speed of 300 ± 30 mm/min. After the peeling has been started, the average value of a force within a period from the instant when 10 mm was peeled to the instant when 40 mm was peeled is found with an integrator. The measurements are conducted for five samples and the average of those five values is considered as the above-mentioned peeling force.

Further, in the auxiliary implement for patch application in accordance with the present invention, the bending resistance of the auxiliary implement measured based on the 45° cantilever method specified by JIS L 1086-1983 is preferably 5-30 cm. In accordance with the present invention, "bending resistance" indicates the bending resistance [cm] representing the bending resistance of a sample (2 x about 15 cm) measured based on the 45° cantilever method specified by JIS L 1086-1983 6.12.1.

Integrating a patch with the auxiliary implement having a bending resistance of 5-30 cm makes it possible to adjust the strength or flexibility thereof more accurately to the level allowing for easy handling during application operation. As a result, the patch can be smoothly carried to the desired application area, while preventing the problem of partial mutual bonding of the adhesive surfaces of the patch caused by the patch bending during the application operation, and the patch can be applied more reliably without wrinkles.

Furthermore, if the bending resistance of the auxiliary implement is 5-30 cm, as described above, the ability to resist bending (flexibility) of the auxiliary implement can be easily adjusted to a level allowing the end portion of the auxiliary implement to be peeled from the rear surface of the patch backing by lifting therefrom when the patch and auxiliary implement are integrated and bent. Therefore, when the auxiliary implement is peeled off from the patch applied to the affected area, the auxiliary implement alone can be easily peeled off from the patch, while preventing partial or complete peeling of the patch from the affected area by using the repulsion force of the auxiliary implement which causes the return from the curved state to the initial state.

If the bending resistance of the auxiliary implement is less than 5 cm, the ability of the auxiliary implement to resist bending is insufficient and it becomes difficult to prevent the adhesive surfaces of patch from entangling with each other during application, the appearance of wrinkles in the applied patch, and partial or complete peeling of the patch from the affected area. On the other hand, if the bending resistance of the auxiliary implement exceeds 30 cm, the ability of the auxiliary implement to resist bending increases, the adhesive surface of the patch is difficult to deform along the curved surface of the affected area having irregularities when a patch integrated with the auxiliary implement is used for applying to the affected area, and the patch is difficult to apply smoothly and reliably to the affected area. From the standpoint similar to the above-described one, it is more preferred that the bending resistance of the auxiliary implement be 7-22 cm.

Further, in the auxiliary implement for patch application in accordance with the present invention, from the standpoint similar to that described above with respect to the bending resistance of the auxiliary implement, the bending resistance of the product obtained by attaching the patch integrally to the auxiliary implement is preferably 4-25 cm, more preferably 6-18 cm.

### Brief Description of the Drawings

FIG. 1 is a cross-sectional view illustrating an embodiment (first embodiment) of the auxiliary implement for patch application in accordance with the present invention;
FIG. 2 is a front view of the auxiliary implement for patch application shown in FIG. 1;
FIG. 3 is a cross-sectional view illustrating a case in which a peeling sheet is attached to the auxiliary implement for patch application shown in FIG. 1;
FIG. 4 is a cross-sectional view illustrating a state in which a patch is attached to the auxiliary implement for patch application shown in FIG. 1;
FIG. 5 is a cross-sectional view illustrating another embodiment (second embodiment) of the auxiliary implement for patch application in accordance with the present invention;
FIG. 6 is a front view, as viewed from the rear surface of the backing, of the auxiliary implement for patch application shown in FIG. 5;
FIG. 7 is a front view, as viewed from the patch holding surface, of the auxiliary implement for patch application shown in FIG. 5;
FIG. 8 is a cross-sectional view illustrating a state in which a patch is attached to the auxiliary implement for patch application shown in FIG. 5;
FIG. 9 is a front view illustrating yet another embodiment of the auxiliary implement for patch application in accordance with the present invention;
FIG. 10 is a front view illustrating yet another embodiment of the auxiliary implement for patch application in accordance with the present invention;
FIG. 11 is a front view, as viewed from the rear surface of the backing, illustrating yet another embodiment of the auxiliary implement for patch application in accordance with the present invention;
FIG. 12 is a front view, as viewed from the patch holding surface, illustrating the auxiliary implement for patch application shown in FIG. 11; and
FIG. 13 is a front view illustrating yet another embodiment of the auxiliary implement for patch application in accordance with the present invention.

### Best Modes for Carrying Out the Invention

The preferred embodiments of the auxiliary implement for patch application in accordance with the present invention will be described below in greater details with reference to the appended drawings. In the drawings, identical or equivalent components are assigned with the same symbols and redundant explanation is avoided.

### [First Embodiment]

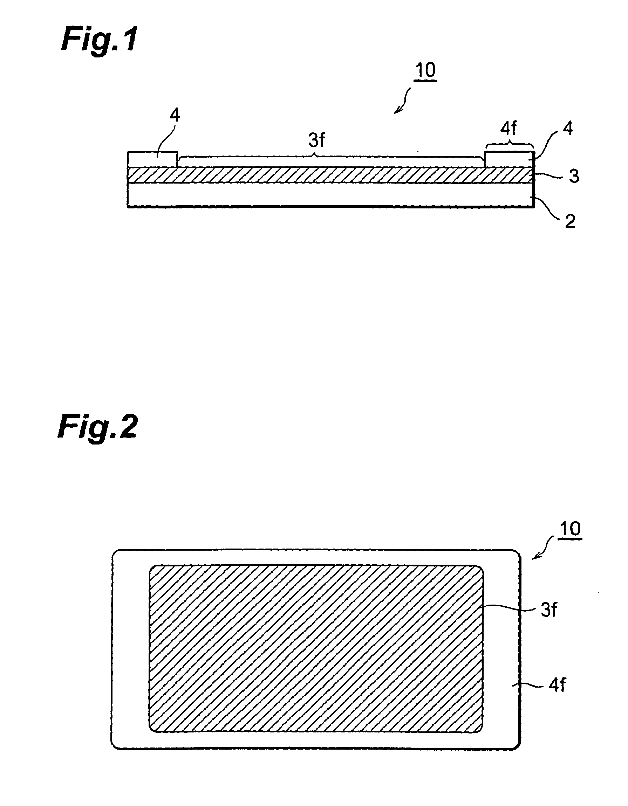

FIG. 1 is a cross-sectional view illustrating the basic configuration of the first embodiment of the auxiliary implement for patch application in accordance with the present invention. FIG. 2 is a front view of the auxiliary implement 10 for patch application shown in FIG. 1.

As shown in FIG. 1 and FIG. 2, the auxiliary implement 10 for patch application in accordance with the present invention (hereinbelow referred to as "auxiliary implement 10") is composed of a rectangular backing 2 having a surface larger than that of the patch which is to be used, an adhesive layer 3 laminated on one surface of the backing 20, and a non-adhesive coating material 4 laminated on the adhesive layer 3 so as to cover the entire outer edge portion of the upper surface of adhesive layer 3. Further, as shown in FIG. 2, in case of such an auxiliary implement 10, the surface of backing 2 where the adhesive layer 3 was laminated thereon is a patch holding surface for temporarily and peelably holding the patch, an upper surface 4f of coating material 4 functions as a non-adhesive surface portion, and a portion 3f of the upper surface of adhesive layer 3 which is not covered with non-adhesive surface portion 4f functions as an adhesive surface portion.

The above-described structural elements will be described below in greater detail. Backing 2 is a sheet with a smooth surface. No limitation is placed on the size of the backing 2, provided that it is larger than the patch which is to be used. No limitation is also placed on the shape of backing 2, but because the patches that are usually used have a rectangular shape, it is preferred that the backing have a rectangular shape to facilitate positioning of the patch with respect to the application area and to facilitate determining the orientation of the patch in the application area.

Further, backing 2 preferably has an appropriate strength (rigidity) and appropriate flexibility to support the patch that will be integrated therewith, to compensate for insufficient strength or flexibility of the patch, and to allow for smooth application operation. As a result, even when a thin patch is used and when some irregularities are present in the vicinity of the application area, the patch holding surface of backing 2 will be deformed following those irregularities and, therefore, the patch will be fittingly applied to the application area. Further, the strength (rigidity) of backing 2 is preferably such that when the end portions of backing 2 are grasped and laid down, an almost parallel state is maintained without curving. In particular, as described above, it is even more preferred that the bending resistance of backing 2 itself be adjusted so that the bending resistance at the time the auxiliary implement 10 is assembled becomes 5-30 cm.

Integrating a patch with the auxiliary implement 10 comprising the backing with such a strength (or bending resistance) allows the strength or flexibility thereof to be adjusted to a level providing for easy handling during application operation. Therefore, the patch can be smoothly carried to the desired application area, while preventing the problem of partial bonding of the adhesive surfaces of the patch to each other caused by the patch bending during application operation. Moreover, the patch can be evenly applied without wrinkles.

Further, as described above, the bending resistance of the structure obtained by attaching a patch to and integrating with the auxiliary implement 10 is preferably adjusted to 4-25 cm. In such a case, when auxiliary implement 10 is peeled off from the patch applied to the affected area, only auxiliary implement 10 can be easily peeled off from the patch, while preventing partial or complete peeling of the patch from the affected area by using the repulsion force of auxiliary implement 10 which causes the return from the curved state to the initial state.

Examples of suitable structural materials of backing 2 include films or sheets of polyethylene, polyethylene terephthalate, polyamide, polyvinyl alcohol, polyvinyl chloride, and the like, paper, foils, nonwoven fabrics, silicon-processed paper, and the like. Furthermore, the advantageous thickness of backing 2 is determined according to the selected structural material and adjusted so as to provide the formed backing 2 with sufficient flexibility and strength allowing it to support a patch.

The adhesive layer 3 is formed by coating or printing a pressure-sensitivity adhesive on one surface of backing 2. This pressure-sensitive adhesive has a pressure-sensitive pseudoadhesive capability allowing the patch to be attached to and detached from the rear surface of the backing. Examples of suitable constituents of the adhesive include acrylic resins such as acrylic acid, acrylic acid esters, 2-ethylhexyl acrylate, n-butyl acrylate, isobutyl acrylate, hexyl acrylate, octyl acrylate, isooctyl acrylate, decyl acrylate, isodecyl acrylate, lauryl acrylate, stearyl acrylate, and the like, ethylene - vinyl acetate copolymer, isoprene, styrene - isoprene - styrene copolymer, polyisobutylene, and the like. The adhesives manufactured from the acrylic resins are especially preferred because they can maintain a constant adhesive strength even after multiple repeated attachment of the patch to the rear surface of the backing and detachment therefrom.

Furthermore, surface irregularities can be formed on the adhesive coated surface by introducing fine particles of silica, kaolin, talk, aluminum hydroxide, titanium oxide, zinc oxide, clay, and the like into the pressure-sensitive adhesive constituting the adhesive layer 3. In such a case, the adhesion between the patch holding surface and the rear surface of the patch backing is point adhesion provided by the surface irregularities, the adhesive strength can be controlled more accurately, and the auxiliary implement 10 can be peeled off more easily from the patch after the patch has been applied to the affected area. To provide for balance with the practical thickness of adhesive layer 3, it is preferred that the particle size of those fine particles be 1-30 µm.

Because of the above-described considerations, the adhesive strength of the upper surface portion 3f of adhesive layer 3 that serves as an adhesive surface portion of the patch holding surface of auxiliary implement 10 is adjusted to a range of 1.7-4.0N as a value based on the measurement test according to the probe tack test specified by JIS Z 0237-1991, and the peeling force acting between the rear surface of the patch backing and the upper surface portion 3f of adhesive layer 3 is adjusted to a range of no more as 1.0 N/20 mm as a value based on the measurement test according to the 180-degree peeling test specified by JIS Z 0237-1991. Such auxiliary implement 10 makes it possible to prevent a problem associated with peeling of part or entire patch from the affected area when auxiliary implement 10 is peeled off from the patch after application to the affected area, and a sufficient bonding strength of the upper surface portion 3f of adhesive layer 3 is maintained even after the repeated usage of the auxiliary implement with a large number of patches.

For example, silicon-processed paper, polypropylene film and polyethylene terephthalate film subjected to silicon processing, and the like can be used as the coating material 4. When this auxiliary implement 10 is used, the application operation can be smoothly conducted, without paying special attention, by holding the non-adhesive upper surface portion 4f of coating material 4 between the fingers during utilization.

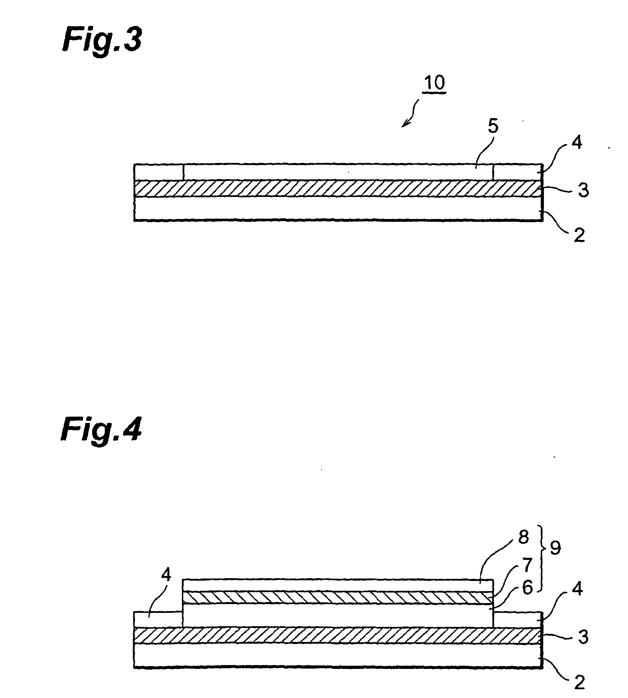

Further, as shown in FIG. 3, a peeling sheet 5 for protecting the upper surface portion 3f of adhesive layer 3 serving as the adhesive surface portion of the patch holding surface may be provided in such auxiliary implement 10. Similarly to coating material 4, for example, silicon-processed paper, polypropylene film and polyethylene terephthalate film subjected to silicon treatment, and the like can be used for the peeling sheet 5. In such case, when the auxiliary implement 10 is stored, no dust adheres to the upper surface portion 3f of adhesive layer 3 serving as the adhesive surface portion of the patch holding surface. Therefore, degradation of the adhesive strength can be effectively prevented and the number of times of repeated usage can be increased.

No specific limitation is placed on the patch with which the auxiliary implement 10 can be employed. However, in order to integrate the entire patch with auxiliary implement 10 and to cause auxiliary implement 10 to demonstrate fully the above-described functions thereof, it is preferred that the size of the patch be less than that of auxiliary implement 10. It is even more preferred that the patch have a surface area less than that of the upper surface portion 3f of adhesive layer 3, which allows the entire patch to be completely attached to the upper surface portion 3f of adhesive layer 3.

From the standpoint of using the auxiliary implement 10 under the conditions such that the adhesive strength of upper surface portion 3f of adhesive layer 3 serving as the adhesive surface portion (a value based on the measurement test according to the probe tack test specified by JIS Z 0237-1991) the peeling force acting between the rear surface of the patch backing and the upper surface portion 3f of adhesive layer 3 (a value based on the measurement test according to the 180 degree peeling test specified by JIS Z 0237-1991), and the bending resistance of the product obtained by attaching the patch integrally to auxiliary implement 10 (a value measured based on 45° cantilever method specified by JIS L 1086-1983) are fit within the above-described numerical ranges, it is preferred that non-stretchable or stretchable materials such as woven fabric, non-woven fabric, knitted cloth, and the like, for example, composed of polyethylene terephthalate (PET), polypropylene, polybutadiene, Nylon, polyurethane, rayon, and the like be used as the structural material of the patch backing. Highly stretchable polyethylene terephthalate knitted cloth is especially preferred because it can closely adhere to the skin and causes little irritation of the skin, the fibers hardly adhere to the upper surface portion 3f of adhesive layer 3 of auxiliary implement 10, and the auxiliary implement 10 can be used any number of times.

A method for using the auxiliary implement 10 of the present embodiment illustrated by FIG. 1 and FIG. 2 will be explained below based on FIGS. 1 through 4.

First, as shown in FIG. 3, when peeling sheet 5 is attached to auxiliary implement 10, the end portion of peeling sheet 5 is peeled off from the upper surface portion 3f of adhesive layer 3 of auxiliary implement 10 by curving the auxiliary implement 10, the peeling sheet is then peeled off by holding the end portion between the fingers, and a state shown in FIG. 1 and FIG. 2 is attained. Then, as shown in FIG. 4, the patch 9 which is to be used is set on auxiliary implement 10. At this time, the patch is spread and set evenly and without wrinkles, and held so that the rear surface side of backing 6 of patch 9 faces the upper surface portion 3f of adhesive layer 3 of auxiliary implement 10.

The patch 9 is thus spread and set evenly and without wrinkles on auxiliary implement 10 before the patch 9 is directly applied to the affected area. Therefore, it is possible to prevent the portions of the surface of adhesive agent layer 7 which contains medically effective components from adhering to each other because of patch 9 bending during the application operation.

A peeling sheet 8 of patch 9 is then peeled off. Auxiliary implement 10 integrated with patch 9 is held between the fingers and carried to the affected area. If at this time the non-adhesive upper surface 4f of coating material 4 of auxiliary implement 10 is held between the fingers, the application operation can be conducted without the adhesion of the surface of adhesive agent layer 7 of patch 9 to the finger tips. The surface of adhesive agent layer 7 of patch 9 is then positioned above the affected area, the application position is fixed, and the patch is applied. At this time, the rear surface of backing 2 of auxiliary implement 10 is lightly pushed in toward the affected area.

Because backing 2 of auxiliary implement 10 has appropriate flexibility, backing 2 is thus slightly curved toward the affected area and spreads radially and outwardly over the affected area, while applying pressure to the periphery of the affected area. As a result, the irregular surface of the affected area becomes an almost smooth surface, the central part thereof is brought in contact with the central part of the surface of adhesive agent layer 7 of patch 9, and the entire patch 9 is gradually and evenly pasted onto the affected area surface. If the size of auxiliary implement 10 is set larger than that of patch 9, then patch 9 is fully pasted and fixed not only to the central part of the affected area, but also to the entire outer edge. Therefore, the problem of the outer edge peeling after application does not occur.

The auxiliary implement 10 is then, for example, curved, the end part thereof is held between the fingers, and auxiliary implement 10 is pulled up and separated from the affected area. At this time, because the adhesive strength of adhesive surface portion of patch holding surface was adjusted to meet the above-described condition, the adhesive strength of adhesive agent layer 7 of patch 9 with respect to the affected area is greater than the adhesive strength of the adhesive surface portion of the patch holding surface of auxiliary implement 10 with respect to patch 9, and the patch is easily and rapidly peeled off from auxiliary implement 10. Though auxiliary implement 10 is separated, the patch 9 remains deformed following the wavy surface of the affected area and fits evenly without wrinkles due to flexibility of backing 6 thereof. In case the auxiliary implement is stored after usage, the peeling sheet 5 is again set on the patch holding surface. In the absence of peeling sheet 5, for example, non-adhesive paper replacing the peeling sheet 5 may be used or the auxiliary implement may be inserted in a commercial vinyl bag.

When the bending resistance measured based on the 45° cantilever method specified by JIS L 1086-1983 is adjusted to 5-30 cm, after patch 9 has been applied to the affected area, patch 9 fits on the curved surface of the affected area. By contrast, because auxiliary implement 10 has the above-described ability to resist bending, it assumes a state in which a repulsion force acts so as to return the auxiliary implement from the curved state to the original state and the end portion of the patch holding surface of backing 2 floats above the rear surface of backing 6 of patch 9 and can be easily peeled off, or a state in which the end portion of the patch holding surface of backing 2 has already been peeled off. Lightly touching this end portion with a finger can increase the peeled portion of the end portion of the patch holding surface of backing 2 so that it can be held between the fingers and the auxiliary implement 10 can be pulled up and separated from the affected area by holding this portion between the fingers.

Auxiliary implement 10 for patch application of the present embodiment thus makes it possible to apply patch 9 smoothly and evenly to the affected area.

### [Second Embodiment]

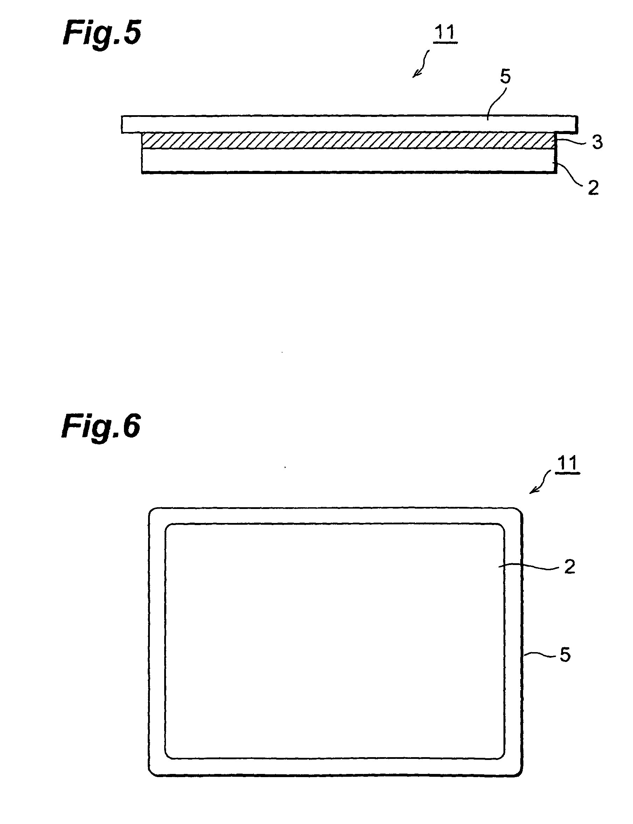

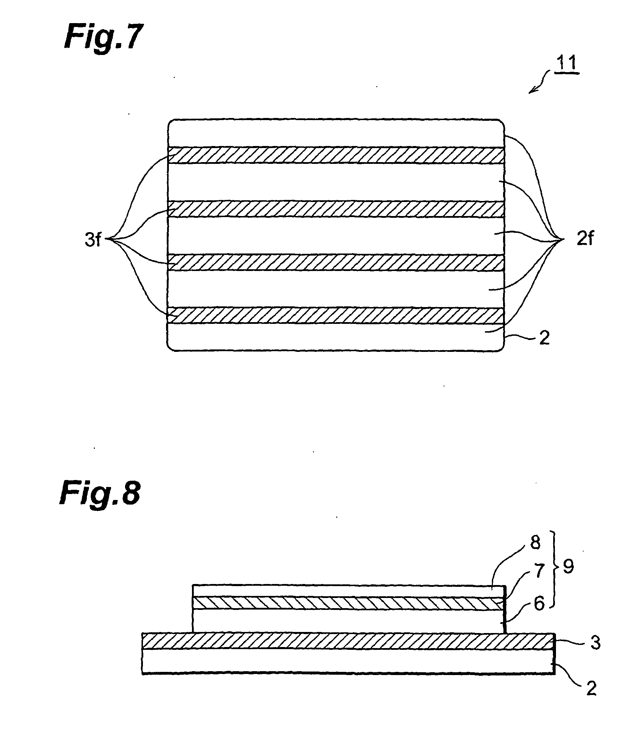

The second embodiment of the auxiliary implement for patch application in accordance with the present invention will be described below with reference to FIGS. 5, 6, and 7. The elements identical to the elements explained with respect to the above-described first embodiment are assigned with the same reference symbols and the redundant explanation thereof is omitted. FIG. 5 is a cross-sectional view illustrating the basic structure of the second embodiment of the auxiliary implement for patch application in accordance with the present invention. FIG. 6 is a front view, as viewed from the rear surface of the backing, of the auxiliary implement 11 for patch application shown in FIG. 5. FIG. 7 is a front view, as viewed from the patch holding surface, of auxiliary implement 11 for patch application shown in FIG. 5.

As shown in FIG. 5, auxiliary implement 11 for patch application of the present embodiment (hereinbelow referred to as "auxiliary implement 11") is mainly composed of a rectangular backing 2 having a surface larger than that of the patch which is to be used and an adhesive layer 3 laminated on one surface of backing 2. Further, as explained with reference to auxiliary implement 10 of the first embodiment, a peeling sheet can be attached, if necessary, to protect the adhesive surface portion of adhesive layer 3. Composed of a non-adhesive coating material 4 laminated on adhesive layer 3 so as to cover the entire outer edge portion of the upper surface of adhesive layer 3.

In case of such auxiliary implement 11, adhesive layer 3, as shown in FIG. 7, is formed in the shape of stripes on the upper surface of backing 2 and portions 3f formed in the shape of stripes function as adhesive surface portions . Portions 2f that were not laminated with adhesive layer 3 and are formed in the shape of stripes also on the upper surface of adhesive layer 3 by portions 3f formed in the shape of stripes function as non-adhesive surface portions.

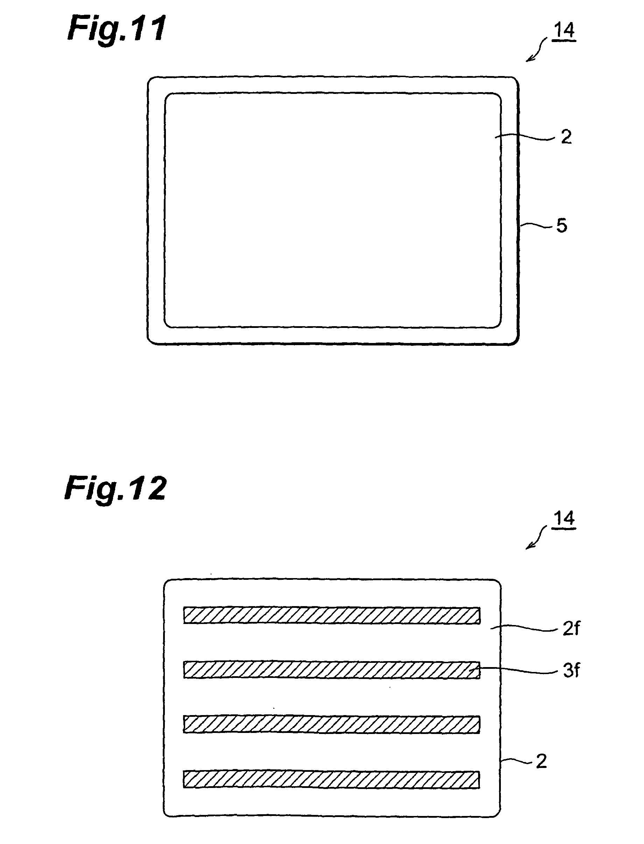

As described above, stripe-like adhesive layer 3 is provided on the upper surface of backing 2 so that at least part of the outer edge portion thereof becomes a portion 2f serving as a non-adhesive surface portion, as shown in FIG. 11, in order to facilitate handling of auxiliary implement 11 after integration of the patch, when the application operation is conducted. Furthermore, taking into account that the auxiliary implement 11 can be handled by an elderly person, for example, the portion 2f that functions as a non-adhesive surface portion which is easy to hold between the fingers during application operation may be colored to point it up visually to the elderly person.

Thus, in auxiliary implement 11 in which the adhesive surface portion and non-adhesive surface portion have a stripe-like shape, as described above, the adhesive surface portion and non-adhesive surface portion can be orderly arranged on the patch holding surface. Therefore, portions that adhere closely between the rear surface of the patch backing and the patch holding surface and those that do not adhere closely are formed with good balance.

From the above-described standpoint, the ratio of the surface area of the adhesive surface portion to the entire surface area of patch holding surface, that is, in the present case, the ratio of the surface area of portion 3f of adhesive surface portion 3 formed in a stripe-like fashion to the entire surface area of the upper surface of backing 2 is preferably adjusted so as to be 20-40%, and the adhesive strength between the patch holding surface and the rear surface of the patch backing is adjusted to a level at which it can reliably hold the patch on the patch holding surface prior to application to the affected area and at which the auxiliary implement can be smoothly peeled off from the patch after application to the affected area.

Further, from the above-described standpoint, the adhesive strength of upper surface portion 3f of stripe-like adhesive layer 3 serving as an adhesive surface portion of the patch holding surface of auxiliary implement 11 is preferably adjusted to a range of 4.0-7.5 N, as a value based on the measurement test according to the probe tack test specified by JIS Z 0237-1991. Furthermore, the peeling force acting between the rear surface of the patch backing and upper surface portion 3f of stripe-like adhesive layer 3 is preferably adjusted to a range of no more than 1.0 N/20 mm, as a value based on the measurement test according to the 180-degree peel method specified by JIS Z 0237-1991.

Here, as described above, the bending resistance of backing 2 itself is preferably adjusted so that the bending resistance measured based on the 45° cantilever method specified by JIS L 1086-19834 during assembly of auxiliary implement 10 is 5-30 cm. Further, as described above, it is also preferred that the bending resistance of the product obtained by attaching the patch integrally to auxiliary implement 10 be adjusted to 4-25 cm.

Accordingly, auxiliary implement 11 makes it possible to prevent the occurrence of the problem associated with partial or complete peeling of the patch from the affected area when auxiliary implement 11 is peeled off from the patch after application to the affected area. Moreover, a sufficient adhesive strength of upper surface portion 3f of stripe-like adhesive layer 3 can be maintained even in repeated usage with a large number of patches.

Furthermore, the width of the spacing between the adjacent stripes in upper surface portion 3f of stripe-like adhesive layer 3 is preferably 12-16 mm. In such a case, adhesive surface portions can be arranged in a uniform and orderly manner on the upper surface of backing 2, without local shift of the pressure-sensitive adhesive to one side, and the adhesive strength of patch holding surface can be made more uniform over the entire rear surface of the patch backing.

Further, surface irregularities may be formed on the adhesive coating surface by introducing fine particles of silica, kaolin, talk, aluminum hydroxide, titanium oxide, zinc oxide, clay, and the like in the pressure-sensitive adhesive constituting adhesive layer 3. In such a case, the adhesion between the adhesive surface of the patch holding surface and the rear surface of the patch backing is point adhesion provided by the surface irregularities, the adhesive strength can be controlled more accurately, and auxiliary implement 11 can be peeled off more easily from the patch after the patch has been applied to the affected area. To provide for balance with the practical thickness of adhesive layer 3, it is preferred that the particle size of those fine particles be 1-30 µm.

A method for using auxiliary implement 11 of the present embodiment illustrated by FIG. 5 will be briefly described below with reference FIGS. 5 through 8. First, as shown in FIG. 5, when peeling sheet 5 is attached to auxiliary implement 11, the end portion of peeling sheet 5 is peeled off from upper surface portion 3f of adhesive layer 3 of auxiliary implement 11 by curving auxiliary implement 11, the peeling sheet is then peeled off by holding the end portion between the fingers, and a state shown in FIG. 7 is assumed. Then, as shown in FIG. 8, the patch 9 which is to be used is set on auxiliary implement 11 by spreading it evenly, without wrinkles. Then, the patch 9 is applied to the affected area as with the auxiliary implement 10 of the above-described first embodiment.

The preferred embodiments of the present invention were described above in details, but the present invention is not limited to those embodiments.

For example, in the auxiliary implement for patch application in accordance with the present invention, when the adhesive surface portions and non-adhesive surface portions have a stripe-like shape, no limitation is placed on the specific shape of the stripes, and in addition to a linear shape such as shown in auxiliary implement 11 of the second embodiment, the stripes may have a wave-like shape or a combination of linear and wave-like shapes.

Moreover, in auxiliary implement 11 of the above-described second embodiment, the explanation was conducted with respect to a stripe-like shape of adhesive surface portions or non-adhesive surface portions. However, in the auxiliary implement for patch application in accordance with the present invention, no specific limitation is placed on the shape of adhesive surface portions or non-adhesive surface portions. For example, a grid-like or spot-like shape may be used. Examples of specific shapes suitable for a grid-like configuration include square, rectangular, and rhomboidal shapes. Example of specific shapes suitable for a spot-like configuration include round, square, rectangular, rhomboidal, and elliptical shapes.

Furthermore, in auxiliary implement 10 of the above-described first embodiment, the explanation was conducted with respect to a configuration in which adhesive layer 3 was coated over the entire upper surface of backing 2. However, when coating material 4 is attached to the outer edge portion of the upper surface of backing 2, adhesive layer 3 may be also formed in a stripe-like fashion on the upper surface of backing 2, similarly to auxiliary implement 11 of the second embodiment.

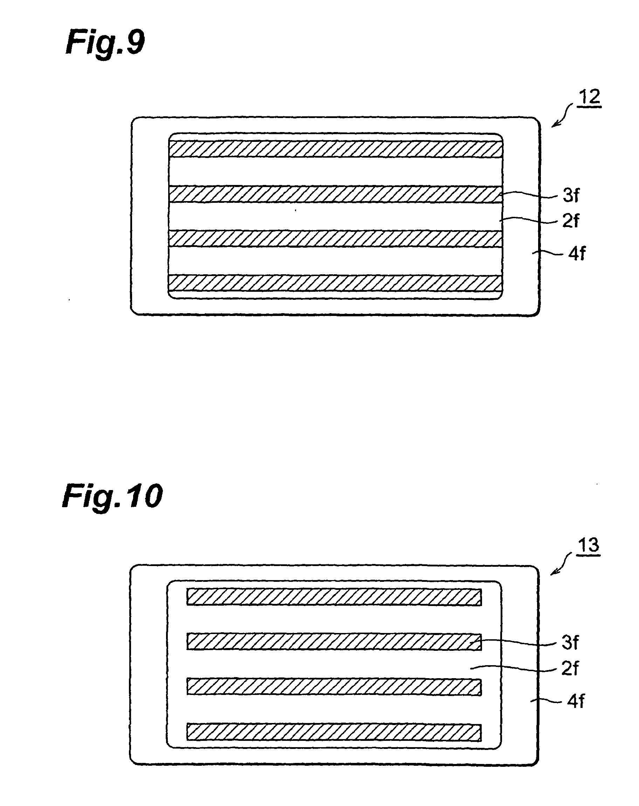

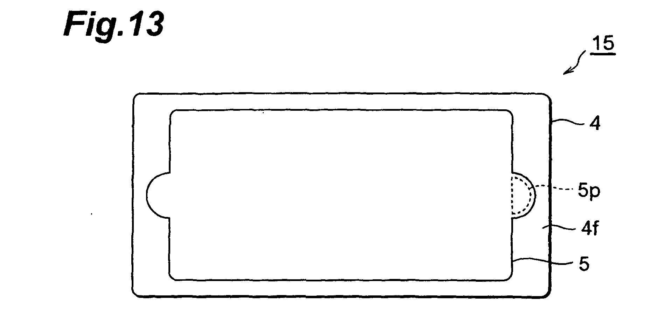

Examples of such auxiliary implements include an auxiliary implement 12 having a configuration shown in FIG. 9 and an auxiliary implement 13 having a configuration shown in FIG. 13. In case of auxiliary implement 12 shown in FIG. 9, portions 3f of the upper surface of adhesive agent layer 2 formed in a stripe-like fashion, which are not coated with coatingmaterial 4, function as the adhesive surface portion of the patch holding surface, and the upper surface 4f of coating material 4 and the portions 2f of the upper surface of the backing, which are not coated with adhesive layer 3 and coating material 4, function as the adhesive surface portions of the patch holding surface. Further, in case of auxiliary implement 13 shown in FIG. 10, the upper surface portion 3f of adhesive agent layer 2 formed in a stripe-like fashion functions as the adhesive surface portion of the patch holding surface, and the upper portion 4f of coating material 4 and portion 2f of the upper surface of the backing, which is not coated with adhesive layer 3, function as non-adhesive surface portions.

Further, in auxiliary implement 11 of the above-described second embodiment, the explanation was conducted with respect to a case in which stripe-like adhesive layer 3 formed on the upper surface of backing 2 was formed from the end portion of one of a pair of mutually opposing sides on the outer edge of the upper surface of backing 2 to the end portion of the other side. However, stripe-like adhesive layer 3 may be also formed so that the entire outer edge portion of the upper surface of backing 2 serves as a non-adhesive surface portion, as in an auxiliary implement 14 shown in FIGS. 11 and 12.

Further, in the auxiliary implement for patch application in accordance with the present invention, when a peeling sheet is attached to the auxiliary implement, no specific limitation is placed on the shape of the peeling sheet. For example, protruding holding portions 5p may be provided on the outer edge portion of peeling sheet 5 to facilitate attachment and detachment of peeling sheet 5, as in an auxiliary implement 15 shown in FIG. 13. In auxiliary implement 15 shown in FIG. 13, two holding portions 5p are provided, but when protruding holding portions are provided on the outer edge portion of peeling sheet in the auxiliary implement for patch application in accordance with the present invention, no specific limitation is placed on the number of holding portions, and at least one holding portion may be provided.

### Examples

The essence of the auxiliary implement for patch application in accordance with the present invention will be described hereinbelow in greater detail with reference to examples thereof and comparative examples. However, those examples of the present invention place no limitation thereon.

For convenience of the following explanation of the examples and comparative examples of auxiliary implements, structural elements identical or equivalent to those of auxiliary implement 10 and auxiliary implement 12 shown in FIG. 3 and FIG. 9 will be assigned with the same symbols.

Further, the "adhesive strength" described hereinbelow indicates the adhesive strength of the adhesive surface portion based on the measurement test according to the probe tack test specified by the aforementioned JIS Z 0237-1991. Furthermore, the "bending resistance"' described hereinbelow indicates a value representing the ability to resist bending of the auxiliary implement measured based on the 45° cantilever method specified by the aforementioned JIS L 1086-1983. Furthermore, the "peeling force" described hereinbelow indicates the value representing the peeling force acting between the rear surface of the patch backing and the adhesive surface portion of the auxiliary implement, which is measured based on the measurement test according to the 180-degree peeling method specified by the aforementioned JIS Z 0237-1991.

First, an auxiliary implement 10 with a bending resistance (upon removal of peeling sheet 5 and coating material 4) of 5-30 cm and a bending resistance (upon removal of peeling sheet 5 and coating material 4) of the product obtained by attaching a patch 9 integrally to auxiliary implement 10 of 4-25 cm was fabricated as auxiliary implement 10 of the below-described Examples 1 through 13 and

### Comparative Example 1.

A patch "La Salonpas" (trade name, manufactured by Hisamitsu Pharmaceuticals Co., Ltd.) comprising a stretchable knitted cloth consisting of polyethylene phthalate (PET) as backing 6 was used as patch 9 (100 mm x 70 mm) used together with auxiliary implement 10 of the below-described Examples 1 through 13 and Comparative Example 1. The bending resistance of patch 9 was 1.5 cm.

### (Example 1)

An auxiliary implement having a structure identical to that of auxiliary implement 10 shown in FIG. 3 was fabricated in the manner as follows . First, high-grade paper (weight 90 g/m², thickness 0.12 mm) was prepared as a backing 2 and an acrylic pressure-sensitive adhesive was coated on the entire surface thereof, followed by drying. The composition of the acrylic pressure-sensitive adhesive was adjusted so that the adhesive strength during drying of the patch holding surface formed on the upper surface of backing 2 was 3.5 N. A paper coating material 4 was then coated on the patch holding surface, followed by cutting to a size of 150 mm x 80 mm. The central part of coating material 4 was then half cut and this portion was used as a peeling sheet 5. The size (100 mm x 70 mm) of the portion serving as the half-cut peeling sheet 5 was identical to that of a patch 9 which is to be used.

The bending resistance (upon removal of peeling sheet 5 and coating material 4) of the auxiliary implement 10 thus obtained was 8 cm. The bending resistance (upon removal of peeling sheet 5 and coating material 4) of the product obtained by attaching patch 9 integrally to auxiliary implement 10 was 6 cm. The peeling force acting between the rear surface of backing 6 of patch 9 and the adhesive surface portion of auxiliary implement 10 was 0.3 N/20 mm.

### (Example 2)

An auxiliary implement having a structure identical to that of auxiliary implement 12 shown in FIG. 9 was fabricated in the manner as follows. First, high-grade paper identical to that of Example 1 was prepared as a backing 2 and an acrylic pressure-sensitive adhesive was coated on the surface thereof in a stripe-like fashion (width of stripes serving as adhesive surface portions: 6 mm, width of stripes serving as non-adhesive surface portions: 14 mm, the ratio of the surface area of adhesive surface portions to the entire surface area of the patch holding surface: 30%), followed by drying. The composition of the acrylic pressure-sensitive adhesive was adjusted so that the adhesive strength during drying of the patch holding surface formed on the upper surface of backing 2 was 6.0 N. Then, processing was conducted in the same manner as in Example 1 and an auxiliary implement was fabricated which had a size identical to that in Example 1.

The bending resistance (upon removal of peeling sheet 5 and coating material 4) of the auxiliary implement 10 thus obtained was 10 cm. The bending resistance (upon removal of peeling sheet 5 and coating material 4) of the product obtained by attaching patch 9 integrally to auxiliary implement 10 was 7 cm. The peeling force acting between the rear surface of backing 6 of patch 9 and the adhesive surface portion of auxiliary implement 10 was 0.4 N/20 mm.

### (Example 3)

An auxiliary implement was fabricated which had a structure identical to that in Example 2, except that the width of stripes serving as adhesive surface portions was 8 mm, the width of stripes serving as non-adhesive surface portions was 12 mm, the ratio of the surface area of adhesive surface portions to the entire surface area of the patch holding surface was 40%, and the adhesive strength during drying of the patch holding surface was 4.5 N.

The bending resistance (upon removal of peeling sheet 5 and coating material 4) of the auxiliary implement 10 thus obtained was 9 cm. The bending resistance (upon removal of peeling sheet 5 and coating material 4) of the product obtained by attaching patch 9 integrally to auxiliary implement 10 was 8 cm. The peeling force acting between the rear surface of backing 6 of patch 9 and the adhesive surface portion of auxiliary implement 10 was 0.3 N/20 mm.

### (Example 4)

An auxiliary implement was fabricated which had a structure identical to that in Example 2, except that the adhesive strength during drying of the patch holding surface was 7.5 N and a treatment for coating the adhesive surface by special printing was conducted on a peripheral area of the patch holding surface where the patch is to be applied.

The bending resistance (upon removal of peeling sheet 5 and coating material 4) of the auxiliary implement 10 thus obtained was 12 cm. The bending resistance (upon removal of peeling sheet 5 and coating material 4) of the product obtained by attaching patch 9 integrally to auxiliary implement 10 was 10 cm. The peeling force acting between the rear surface of backing 6 of patch 9 and the adhesive surface portion of auxiliary implement 10 was 0.6 N/20 mm.

### (Example 5)

An auxiliary implement was fabricated which had a structure identical to that in Example 1, except that the adhesive strength during drying of the patch holding surface was 3.09 N.

The bending resistance (upon removal of peeling sheet 5 and coating material 4) of the auxiliary implement 10 thus obtained was 8 cm. The bending resistance (upon removal of peeling sheet 5 and coating material 4) of the product obtained by attaching patch 9 integrally to auxiliary implement 10 was 6 cm. The peeling force acting between the rear surface of backing 6 of patch 9 and the adhesive surface portion of auxiliary implement 10 was 0.3 N/20 mm.

### (Example 6)

An auxiliary implement was fabricated which had a structure identical to that in Example 1, except that the adhesive strength during drying of the patch holding surface was 3.53 N.

The bending resistance (upon removal of peeling sheet 5 and coating material 4) of the auxiliary implement 10 thus obtained was 8 cm. The bending resistance (upon removal of peeling sheet 5 and coating material 4) of the product obtained by attaching patch 9 integrally to auxiliary implement 10 was 6 cm. The peeling force acting between the rear surface of backing 6 of patch 9 and the adhesive surface portion of auxiliary implement 10 was 0.3 N/20 mm.

### (Example 7)

An auxiliary implement was fabricated which had a structure identical to that in Example 1, except that the adhesive strength during drying of the patch holding surface was 3.90 N.

The bending resistance (upon removal of peeling sheet 5 and coating material 4) of the auxiliary implement 10 thus obtained was 9 cm. The bending resistance (upon removal of peeling sheet 5 and coating material 4) of the product obtained by attaching patch 9 integrally to auxiliary implement 10 was 6.5 cm. The peeling force acting between the rear surface of backing 6 of patch 9 and the adhesive surface portion of auxiliary implement 10 was 0.4 N/20 mm.

### (Example 8)

An auxiliary implement was fabricated which had a structure identical to that in Example 1, except that the adhesive strength during drying of the patch holding surface was 1.5 N.

The bending resistance (upon removal of peeling sheet 5 and coating material 4) of the auxiliary implement 10 thus obtained was 7 cm. The bending resistance (upon removal of peeling sheet 5 and coating material 4) of the product obtained by attaching patch 9 integrally to auxiliary implement 10 was 6 cm. The peeling force acting between the rear surface of backing 6 of patch 9 and the adhesive surface portion of auxiliary implement 10 was 0.2 N/20 mm.

### (Example 9)

An auxiliary implement was fabricated which had a structure identical to that in Example 2, except that the adhesive strength during drying of the patch holding surface was 4.1 N.

The rigidity (upon removal of peeling sheet 5 and coating material 4) of the auxiliary implement 10 thus obtained was 9.5 cm. The rigidity (upon removal of peeling sheet 5 and coating material 4) of the product obtained by attaching patch 9 integrally to auxiliary implement 10 was 7 cm. The peeling force acting between the rear surface of backing 6 of patch 9 and the adhesive surface portion of auxiliary implement 10 was 0.3 N/20 mm.

### (Example 10)

An auxiliary implement was fabricated which had a structure identical to that in Example 3, except that the adhesive strength during drying of the patch holding surface was 3.5 N.

The bending resistance (upon removal of peeling sheet 5 and coating material 4) of the auxiliary implement 10 thus obtained was 9 cm. The bending resistance (upon removal of peeling sheet 5 and coating material 4) of the product obtained by attaching patch 9 integrally to auxiliary implement 10 was 7.5 cm. The peeling force acting between the rear surface of backing 6 of patch 9 and the adhesive surface portion of auxiliary implement 10 was 0.3 N/20 mm.

### (Example 11)

An auxiliary implement was fabricated which had a structure identical to that in Example 2, except that the adhesive strength during drying of the patch holding surface was 8.0 N.

The bending resistance (upon removal of peeling sheet 5 and coating material 4) of the auxiliary implement 10 thus obtained was 10 cm. The bending resistance (upon removal of peeling sheet 5 and coating material 4) of the product obtained by attaching patch 9 integrally to auxiliary implement 10 was 7.0 cm. The peeling force acting between the rear surface of backing 6 of patch 9 and the adhesive surface portion of auxiliary implement 10 was 1.2 N/20 mm.

### (Example 12)

An auxiliary implement was fabricated which had a structure identical to that in Example 2, except that the width of stripes serving as adhesive surface portions was 3 mm, the width of stripes serving as non-adhesive surface portions was 17 mm, the ratio of the surface area of adhesive surface portions to the entire surface area of the patch holding surface was 15%, and the adhesive strength during drying of the patch holding surface was 7.5 N.

The bending resistance (upon removal of peeling sheet 5 and coating material 4) of the auxiliary implement 10 thus obtained was 10 cm. The bending resistance (upon removal of peeling sheet 5 and coating material 4) of the product obtained by attaching patch 9 integrally to auxiliary implement 10 was 7.5 cm. The peeling force acting between the rear surface of backing 6 of patch 9 and the adhesive surface portion of auxiliary implement 10 was 1.1 N/20 mm.

### (Example 13)

An auxiliary implement was fabricated which had a structure identical to that in Example 2, except that the width of stripes serving as adhesive surface portions was 10 mm, the width of stripes serving as non-adhesive surface portions was 10 mm, the ratio of the surface area of adhesive surface portions to the entire surface area of patch holding surface was 50%, and the adhesive strength during drying of the patch holding surface was 4.5 N.

The bending resistance (upon removal of peeling sheet 5 and coating material 4) of the auxiliary implement 10 thus obtained was 10 cm. The bending resistance (upon removal of peeling sheet 5 and coating material 4) of the product obtained by attaching patch 9 integrally to auxiliary implement 10 was 7.5 cm. The peeling force acting between the rear surface of backing 6 of patch 9 and the adhesive surface portion of auxiliary implement 10 was 1.3 N/20 mm.

### (Comparative Example 1)

An auxiliary implement was fabricated which had a structure identical to that in Example 1, except that no non-adhesive surface portions were provided on the patch holding surface.

In the auxiliary implement 10 thus obtained, the adhesive strength during drying of the patch holding surface was 1.5 N. The bending resistance (upon removal of peeling sheet 5 and coating material 4) of auxiliary implement 10 was 8.5 cm. The bending resistance (upon removal of peeling sheet 5 and coating material 4) of the product obtained by attaching patch 9 integrally to auxiliary implement 10 was 6 cm. The peeling force acting between the rear surface of backing 6 of patch 9 and the adhesive surface portion of auxiliary implement 10 was 0.2 N/20 mm.

### [Test for evaluating convenience in use (1)]

Twenty users with symptoms of stiffness in a shoulder were asked to use the auxiliary implements for patch application described in Examples 1 through 13 and Comparative Example 1 and the convenience in use was evaluated. The application position of the heretofore explained patch was limited to the affected area of the shoulder. Furthermore, the evaluation items (1)-(5) described below were set as evaluation items for convenience in use, and the users were asked to apply the patch to the same affected area by using three patches for each one auxiliary implement. The users' responses were obtained relating to the test results for evaluation items of those samples based on the following evaluation criteria. Specifically, 2 is given when no problems are associated with evaluation items, and 1 is given when problems are associated with evaluation items. The responses are shown in Table 1. The evaluation results obtained without using the auxiliary implement for patch application are also shown in Table 1. Symbol "-" in the table indicates that evaluation was impossible.

{Evaluation criteria for evaluating the convenience in use} : (1) stability of patch on patch holding surface of the auxiliary implement for patch application (whether or not the patch can be easily carried, without peeling, to the application zone; (2) do the adhesive portions of the patch surface entangle with each other (whether or not the adhesive portions of the patch surface stick to each other during application); (3) state of the patch after application (whether or not the patch can be applied evenly and without wrinkles); (4) handleability of the auxiliary implement during application (whether or not contact of fingers with the adhesive surface of the patch or the adhesive surface of the auxiliary implement allows the application operation to proceed smoothly); (5) peelability of the auxiliary implement after patch application (whether or not the auxiliary implement alone can be smoothly peeled off from the rear surface of the patch backing, without partially or entirely peeling the patch after application).

**Table 1**

| TEST ITEM TEST EXAMPLE | (1) | (2) | (3) | (4) | (5) |
|---|---|---|---|---|---|
| EXAMPLE 1 | 2 | 2 | 2 | 2 | 2 |
| EXAMPLE 2 | 2 | 2 | 2 | 2 | 2 |
| EXAMPLE 3 | 2 | 2 | 2 | 2 | 2 |
| EXAMPLE 4 | 2 | 2 | 2 | 2 | 2 |
| EXAMPLE 8 | 1 | 1 | 1 | 1 | 2 |
| EXAMPLE 9 | 2 | 2 | 2 | 2 | 1 |
| EXAMPLE 10 | 1 | 1 | 1 | 1 | 2 |
| EXAMPLE 11 | 2 | 2 | 2 | 2 | 1 |
| EXAMPLE 12 | 1 | 1 | 1 | 1 | 2 |
| EXAMPLE 13 | 2 | 2 | 2 | 2 | 1 |
| COMPARATIVE EXAMPLE 1 | 1 | 1 | 1 | 1 | 1 |
| NO AUXILIARY IMPLEMENT | - | 1 | 1 | 1 | - |

The results shown in Table 1 clearly confirmed that the auxiliary implements for patch application of Examples 1 through 13 in accordance with the present invention had excellent convenience as compared with that of the auxiliary implement for patch application of Comparative Example 1. In particular, it was confirmed that among the auxiliary implements for patch application in accordance with the present invention, with the auxiliary implements for patch application of Examples 1 through 4, which had the preferred adhesive strength of the adhesive surface portion, the patch could be smoothly carried to the application position and could be applied evenly without wrinkles , while preventing the problems that have been occurring during conventional patch application operations.

### [Test for evaluating by a probe tack test the adhesive strength maintaining ability of the patch holding surface of auxiliary implement in repeated utilization]

One auxiliary implement for patch application of each of Examples 4 through 6 were prepared and the adhesive strength of patch holding surface after 1-15 application operations of each of the auxiliary implement was measured by a probe tack test (the measurement test basically following the probe tack test specified by JIS Z 0237-1991, Section 5.3, except that the contact speed and pull-off speed were 5 mm/sec). The results are shown in Table 2. A patch "La Salonpas" (trade name, manufactured by Hisamitsu Pharmaceuticals Co., Ltd.) comprising a stretchable knitted cloth consisting of polyethylene terephthalate (PET) as a backing was used as the patch.

**Table 2**

| PEELING FREQUENCY | 0 | 1 | 2 | 3 | 5 | 7 | 10 | 15 |
|---|---|---|---|---|---|---|---|---|
| EXAMPLE 5 | 3.03N | 2.38N | 2.25N | 2.36N | 2.41N | 2.32N | 2.11N | 1.97N |
| EXAMPLE 6 | 3.46N | 2.58N | 2.26N | 2.30N | 2.39N | 2.20N | 2.15N | 2.23N |
| EXAMPLE 7 | 3.63N | 2.61N | 2.46N | 2.55N | 2.32N | 2.38N | 2.55N | 2.28N |

The results shown in Table 2 clearly confirmed that in the auxiliary implements for patch application of Examples of 4 through 6 in accordance with the present invention, the value of adhesive strength was maintained above 1.7N, which is the appropriate range, even after using the patch 15 times, which confirmed that the auxiliary implement can be used repeatedly.

Then auxiliary implements 10 with an adhesive strength of 1.5-4.ON were fabricated as auxiliary implements 10 of the below-described Examples 14 through 22 and Comparative Example 2.

A patch 9 used together with the auxiliary implements 10 of the below-described Examples 14 through 22 and Comparative Example 2 was fabricated by using a stretchable knitted cloth consisting of polyethylene phthalate (PET) as a backing 6, preparing an adhesive agent layer 7 by dissolving separately 10 wt. parts of styrene-isobutylene ABA-type block copolymer (SIS), 0.5 wt. part of polybutene (HV300), and 2.5 wt. parts of petroleum resin (Arkon P100) in toluene, mixing, spreading over a peeling liner, and drying, and press bonding the adhesive agent layer to the backing 6. The bending resistance of patch 9 thus obtained was 1.5 cm.

### (Example 14)

An auxiliary implement with the structure identical to that of auxiliary implement 10 shown in FIG. 3 was fabricated in the manner as follows. First, high-grade paper (weight 90 g/m², thickness 0.12 mm) was prepared as backing 2 and a pressure-sensitive adhesive was coated on the entire surface thereof, followed by drying. The composition of the pressure-sensitive adhesive was adjusted so that the adhesive strength during drying of the patch holding surface formed on the upper surface of backing 2 was 3.6 N. The pressure-sensitive adhesive had the following composition: 100 wt. parts of styrene - ethylene - butadiene - styrene block copolymer, 30 wt. parts aliphatic petroleum resin, 0.5 wt. part of liquid terpene resin, and 1 wt. part of antioxidant. The thickness of adhesive layer 3 was 30 µm. The peeling force acting between the rear surface of backing 6 of patch 9 and the adhesive surface portion of auxiliary implement 10 was 0.3 N/20 mm.

A coating material 4 from a silicon-processed paper was then coated on the patch holding surface, followed by cutting to a size of 150 mm x 80 mm. The central part of coating material 4 was then half cut and the respective portion was used as a peeling sheet 5. The size (140 mm x 70 mm) of the portion serving as the half-cut peeling sheet 5 was identical to that of a patch 9 which is to be used. The bending resistance (upon removal of peeling sheet 5 and coating material 4) of the auxiliary implement 10 thus obtained was 6 cm. The bending resistance (upon removal of peeling sheet 5 and coating material 4) of the product obtained by attaching patch 9 integrally to auxiliary implement 10 was 4.5 cm.

### (Example 15)

An auxiliary implement 10 was fabricated which had the structure identical to that in Example 14, except that high-grade paper (weight 150 g/m², thickness 0.2 mm) was used as backing 2 and the adhesive strength during drying of the patch holding surface formed on the upper surface of backing 2 was 2.5 N. The pressure-sensitive adhesive had the following composition: 100 wt. parts of butyl acrylate, 10 wt. parts of acrylic acid, and 1 wt. part of antioxidant. The thickness of adhesive layer 3 was 40 µm. The bending resistance (upon removal of peeling sheet 5 and coating material 4) of the auxiliary implement 10 thus obtained was 15 cm. The bending resistance (upon removal of peeling sheet 5 and coating material 4) of the product obtained by attaching patch 9 integrally to auxiliary implement 10 was 10.4 cm. The peeling force acting between the rear surface of backing 6 of patch 9 and the adhesive surface portion of auxiliary implement 10 was 0.2 N/20 mm.

### (Example 16)

An auxiliary implement 10 was fabricated which had the structure identical to that in Example 14, except that high-grade paper (weight 200 g/m², thickness 0.26 mm) was used as backing 2 and the adhesive strength during drying of the patch holding surface formed on the upper surface of backing 2 was 1.8 N. The pressure-sensitive adhesive had the following composition: 100 wt. parts of butyl acrylate, 10 wt. parts of acrylic acid, and 1 wt. part of antioxidant. The thickness of adhesive layer 3 was 30 µm. The bending resistance (upon removal of peeling sheet 5 and coating material 4) of the auxiliary implement 10 thus obtained was 28 cm. The bending resistance (upon removal of peeling sheet 5 and coating material 4) of the product obtained by attaching patch 9 integrally to auxiliary implement 10 was 23.5 cm. The peeling force acting between the rear surface of backing 6 of patch 9 and the adhesive surface portion of auxiliary implement 10 was 0.2 N/20 mm.

### (Example 17)

An auxiliary implement 10 was fabricated which had the structure identical to that in Example 14, except that high-grade paper (weight 70 g/m², thickness 0.09 mm) was used as backing 2 and the adhesive strength during drying of the patch holding surface formed on the upper surface of backing 2 was 4.0 N. The pressure-sensitive adhesive had the following composition: 100 wt. parts of styrene - ethylene - butadiene - styrene block copolymer, 30 wt. parts of aliphatic petroleum resin, 0.5 wt. part of liquid terpene resin, and 1 wt. part of antioxidant. The thickness of adhesive layer 3 was 40 µm. The bending resistance (upon removal of peeling sheet 5 and coating material 4) of the auxiliary implement 10 thus obtained was 8 cm. The bending resistance (upon removal of peeling sheet 5 and coating material 4) of the product obtained by attaching patch 9 integrally to auxiliary implement 10 was 5.5 cm. The peeling force acting between the rear surface of backing 6 of patch 9 and the adhesive surface portion of auxiliary implement 10 was 0.3 N/20 mm.

### (Example 18)

An auxiliary implement 10 was fabricated which had the structure identical to that in Example 14, except that high-grade paper (weight 150 g/m², thickness 0.2 mm) was used as backing 2 and the adhesive strength during drying of the patch holding surface formed on the upper surface of backing 2 was 1.5 N. The pressure-sensitive adhesive had the following composition: 100 wt. parts of butyl acrylate, 10 wt. parts of acrylic acid, and 1 wt. part of antioxidant. The thickness of adhesive layer 3 was 25 µm. The bending resistance (upon removal of peeling sheet 5 and coating material 4) of the auxiliary implement 10 thus obtained was 25 cm. The bending resistance (upon removal of peeling sheet 5 and coating material 4) of the product obtained by attaching patch 9 integrally to auxiliary implement 10 was 20.5 cm. The peeling force acting between the rear surface of backing 6 of patch 9 and the adhesive surface portion of auxiliary implement 10 was 0.1 N/20 mm.

### (Example 19)

An auxiliary implement 10 was fabricated which had the structure identical to that in Example 14, except that high-grade paper (weight 50 g/m², thickness 0.06 mm) was used as backing 2. The bending resistance (upon removal of peeling sheet 5 and coating material 4) of the auxiliary implement 10 thus obtained was 3 cm. The bending resistance (upon removal of peeling sheet 5 and coating material 4) of the product obtained by attaching patch 9 integrally to auxiliary implement 10 was 2.8 cm. The peeling force acting between the rear surface of backing 6 of patch 9 and the adhesive surface portion of auxiliary implement 10 was 0.4 N/20 mm.

### (Example 20)

An auxiliary implement 10 was fabricated which had the structure identical to that in Example 14, except that high-grade paper (weight 250 g/m², thickness 0.3 mm) was used as backing 2. The bending resistance (upon removal of peeling sheet 5 and coating material 4) of the auxiliary implement 10 thus obtained was 35 cm. The bending resistance (upon removal of peeling sheet 5 and coating material 4) of the product obtained by attaching patch 9 integrally to auxiliary implement 10 was 30.2 cm. The peeling force acting between the rear surface of backing 6 of patch 9 and the adhesive surface portion of auxiliary implement 10 was 0.1 N/20 mm.

### (Example 21)

An auxiliary implement 10 was fabricated which had the structure identical to that in Example 14, except that high-grade paper (weight 50 g/m², thickness 0.06 mm) was used as a backing 2 and the adhesive strength during drying of the patch holding surface formed on the upper surface of backing 2 was 4.5 N. The pressure-sensitive adhesive had the following composition: 100 wt. parts of styrene - ethylene - butadiene - styrene block copolymer, 70 wt. parts of water-added petroleum resin, and 1 wt. part of antioxidant. The thickness of adhesive layer 3 was 30 µm. The bending resistance (upon removal of peeling sheet 5 and coating material 4) of the auxiliary implement 10 thus obtained was 3 cm. The bending resistance (upon removal of peeling sheet 5 and coating material 4) of the product obtained by attaching patch 9 integrally to auxiliary implement 10 was 2.8 cm. The peeling force acting between the rear surface of backing 6 of patch 9 and the adhesive surface portion of auxiliary implement 10 was 0.4 N/20 mm.

### (Example 22)

An auxiliary implement 10 was fabricated which had the structure identical to that in Example 14, except that high-grade paper (weight 250 g/m², thickness 0.3 mm) was used as a backing 2 and the adhesive strength during drying of the patch holding surface formed on the upper surface of backing 2 was 1.5 N. The pressure-sensitive adhesive had the following composition: 100 wt. parts of butyl acrylate, 10 wt. parts of acrylic acid, and 1 wt. part of antioxidant. The thickness of adhesive layer 3 was 25 µm. The bending resistance (upon removal of peeling sheet 5 and coating material 4) of the auxiliary implement 10 thus obtained was 40 cm. The bending resistance (upon removal of peeling sheet 5 and coatingmaterial 4) of the product obtained by attaching patch 9 integrally to auxiliary implement 10 was 35 cm. The peeling force acting between the rear surface of backing 6 of patch 9 and the adhesive surface portion of auxiliary implement 10 was 0.1 N/20 mm.

### (Comparative Example 2)

An auxiliary implement was fabricated which had the structure identical to that in Example 14, except that non-adhesive surface portions were not provided on the patch holding surface. The bending resistance (upon removal of peeling sheet 5 and coating material 4) of the auxiliary implement 10 thus obtained was 6 cm. The bending resistance (upon removal of peeling sheet 5 and coating material 4) of the product obtained by attaching patch 9 integrally to auxiliary implement 10 was 4.5 cm. The adhesive strength during drying of the patch holding surface formed on the upper surface of backing 2 was 4.5N. The peeling force acting between the rear surface of backing 6 of patch 9 and the adhesive surface portion of auxiliary implement 10 was 0.3 N/20 mm.

### [Test for evaluating the convenience in use (2)]

Twenty users with symptoms of stiffness in a shoulder were asked to use the auxiliary implements for patch application described in Examples 14 through 22 and Comparative Example 2 and the convenience in use was evaluated. The application position of the heretofore explained patch was limited to the affected area of the shoulder.

Furthermore, the evaluation items (1)-(5) described below were set as evaluation items for the convenience in use, and the number of grading points relating to those evaluation items was also set as described below. The users were asked to apply the patch to the same affected area by using three patches for each one auxiliary implement and to provide grading relating to those evaluation items for each single application. The grading results (mean values) are shown in Table 3. The evaluation results relating to a case when no auxiliary implement for patch application was used are also shown in Table 3.

{Evaluation criteria for evaluating the convenience in use} : (1) the patch could be easily applied and the auxiliary implement could be easily peeled off : 4 points; (2) the patch could be easily applied, but the auxiliary implement was difficult to peel off : 3 points; (3) the patch was difficult to apply, but the auxiliary implement was easy to peel off : 2 points; (4) the patch was difficult to apply and peeling of the auxiliary implement was also difficult : 1 point.

**Table 3**

| | GRADING VALUES OF EVALUATION ITEMS (1) - (4) (MEAN VALUES) |
|---|---|
| EXAMPLE 14 | 4.0 |
| EXAMPLE 15 | 4.0 |
| EXAMPLE 16 | 4.0 |
| EXAMPLE 17 | 3.5 |
| EXAMPLE 18 | 3.6 |
| EXAMPLE 19 | 2.0 |
| EXAMPLE 20 | 1.9 |
| EXAMPLE 21 | 1.4 |
| EXAMPLE 22 | 1.6 |
| COMPARATIVE EXAMPLE 2 | 2.0 |
| NO AUXILIARY IMPLEMENT | 1.1 |

The results shown in Table 3 clearly confirmed that applying a patch by using the auxiliary implement for patch application of Examples 14 through 22 in accordance with the present invention facilitates the application operation as compared with a case when no auxiliary implement was used and that the auxiliary implement for patch application in accordance with the present invention had excellent convenience.

In particular, it was confirmed that among the auxiliary implements for patch application of Examples 14 through 22, with the auxiliary implements for patch application of Examples 14 through 18, which had the preferred bending resistance of auxiliary implement 10, the patch could be more smoothly carried to the desired application position and could be applied more reliably and without wrinkles, while preventing the problems that have been occurring during the conventional patch application operations, as compared with the auxiliary implement of Comparative Example 2 having the bending resistance of the same order.

### Industrial Applicability

As described above, with the auxiliary implement for patch application in accordance with the present invention the strength and flexibility of the patch are augmented by integrating the auxiliary implement for patch application and the patch. Therefore, the patch can be smoothly carried to the desired application area and can be applied evenly and without wrinkles, while preventing the problem associated with the portions of the adhesive surface of the patch adhering to each other due to bending of the patch during application operation.

Furthermore, because the adhesive strength between the rear surface of the patch and the patch holding surface can be adjusted, the auxiliary implement for patch application can be used repeatedly with a large number of patches, without discarding the auxiliary implement. Moreover, the problems of partial peeling of the patch held on the patch holding surface during application operation or subsequent peeling over the entire surface and separation can be prevented.

## Claims

1. An auxiliary implement for applying a patch which is to be used to the desired area, which comprises a backing having a surface larger than the patch, wherein on one surface of said backing there are provided an adhesive surface portion which is coated with a pressure-sensitive adhesive and serves for temporarily and peelably holding said patch and a non-adhesive surface portion, and at least part of the outer edge portion of said surface constitutes said non-adhesive surface portion.

2. The auxiliary implement for patch application according to claim 1, wherein a detachable peeling sheet for protecting said adhesive surface portion is further provided on said surface.

3. The auxiliary implement for patch application according to claim 1, wherein said adhesive surface portion or said non-adhesive surface portion has a shape of stripes, grid, or spots.

4. The auxiliary implement for patch application according to claim 1, wherein the ratio of the surface area of said adhesive surface portion to the entire surface area of said surface is 20-40%.

5. The auxiliary implement for patch application according to claim 3, wherein the adhesive strength of the adhesive surface portions based on the measurement test according to the probe tack test specified by JIS Z 0237-1991 is 4.0-7.5 N.

6. The auxiliary implement for patch application according to claim 1, wherein the entire outer edge portion of said surface is said non-adhesive surface portion and the entire portion on the inside of said non-adhesive surface portion is said adhesive surface portion.

7. The auxiliary implement for patch application according to claim 6, wherein the adhesive strength of said adhesive surface portion, which is based on the measurement test according to the probe tack test specified by JIS Z 0237-1991, is 1.7-4.0 N.

8. The auxiliary implement for patch application according to claim 1, wherein the peeling force acting between the rear surface of the backing of said patch and said adhesive surface portion, which is measured according to the 180-degree peeling test specified by JIS Z 0237-1991, is no more than 1.0 N/20 mm.

9. An auxiliary implement for patch application, wherein the bending resistance of the auxiliary implement measured based on the 45° cantilever method specified by JIS L 1086-1983 is 5-30 cm.

10. The auxiliary implement for patch application according to claim 9, wherein said bending resistance after said patch has been attached to and integrated with said auxiliary implement is 4-25 cm.
